# EUROPEAN PATENT APPLICATION

(11) **EP 1 977 741 A2**
(43) Date of publication of application: **08.10.2008**
(21) Application number: 08158711.5
(22) Date of filing: 16.03.2005
(51) Int. Cl.: A61K 31/00, A61K 31/165, A61K 45/06, A61P 3/10, A61P 9/12, A61P 9/10, A61P 13/12, A61P 9/00, A61P 39/00, A61P 3/04, A61P 25/06, A61P 35/00

(54) **Use of renin inhibitors in therapy**

(30) Priority: 17.03.2004 US 553877 P; 29.03.2004 US 557358 P
(62) Divisional of application: 05716127.5
(71) Applicant: NOVARTIS AG, 4002 Basel (CH); Novartis Pharma GmbH, 1235 Vienna (AT)
(72) Inventor: Feldman, David, Louis, Teaneck, NJ NJ 07666 (US); Zelenkofske, Steven, Center Valley, PA PA 18034 (US); Dinboeck, Michaela, 4054, Basel (CH); Prescott, Margaret, Forney, Morris Plains, NJ NJ 07950 (US)
(74) Representative: Dietel, Anja

(57) **Abstract**

The present is directed to a method for the prevention of, delay progression to or treatment of a condition or disease selected from diabetes type 2 (associated with or without hypertension), severe hypertension, PH, malignant hypertension, isolated systolic hypertension, familial dyslipidemic hypertension, endothelial dysfunction (with or without hypertension), survival post-MI, increase of formation of collagen and other extracellular matrix proteins, restenosis after stenting, PVD including PAD and peripheral venous disorders, CAD, morbidity, mortality, cerebrovascular diseases, metabolic disorder (Syndrome X), AF, renoprotection, reduction of proteinuria, renal failure, glomerulonephritis, nephrotic syndrome, renal fibrosis, AIN, ATN, acute tubulo-interstitial nephritis, PKD, vascular inflammation, rennin secreting tumors, vasculitides or closure, restenosis of dialysis access grafts comprising administering a compound of formula (I) or a pharmaceutically acceptable salt thereof alone or in combination with another active ingredient.

## Description

Aliskiren inhibits the action of the natural enzyme renin. The latter passes from the kidneys into the blood where it effects the cleavage of angiotensinogen, releasing the decapeptide angiotensin I which is then cleaved in the lungs, the kidneys and other organs to form the octapeptide angiotensinogen II. The octapeptide increases blood pressure both directly by arterial vasoconstriction and indirectly by liberating from the adrenal glands the sodium-ion-retaining hormone aldosterone, accompanied by an increase in extracellular fluid volume. That increase can be attributed to the action of aldosterone. Inhibitors of the enzymatic activity of renin bring about a reduction in the formation of angiotensin I. As a result a proportionately smaller amount of angiotensin II is produced. The reduced concentration of that active peptide hormone is the direct cause of, e.g., the hypotensive effect of renin inhibitors.

Further evaluations revealed that renin inhibitors can be used for a broader range of therapeutic indications.

The invention relates to a method for the prevention of, delay progression to overt to or treatment of a condition or disease selected from:
(a) diabetes type 2 (associated with or without hypertension). For an example of a model useful to demonstrate the treatment of renal protection in type 2 diabetes with HT, see Kelly et al., Kid Int, Vol. 54, pp. 343-352 (1998); and for renal protection DM w/o HT: db/db mice, see Ziyadeh et al., Proc Natl Acad Sci U S A, Vol. 97, No. 14, pp. 8015-8020 (2000);
(b) severe hypertension, pulmonary hypertension (PH), malignant hypertension, isolated hypertension and familial dyslipidemic hypertension. For an example of a model useful to demonstrate the treatment of severe hypertension and malignant hypertension, see Park et al., Am J Hypertens, Vol. 15, No. 1, Part 1, pp. 78-84 (2002); and for PH, see Jones et al., Am J Physiol Heart Circ Physiol (2004);
(c) endothelial dysfunction (with or without hypertension). For an example of a model useful to demonstrate the treatment of endothelial dysfunction (with or without hypertension), see Shinozaki et al., Diabetes, Vol. 48, pp. 2437-2445 (1999);
(d) survival post-myocardial infarction (MI); increase of formation of collagen and other extracellular matrix proteins and coarctation of aorta. For an example of a model useful to demonstrate the treatment of survival post-MI and increase of formation of collagen, see Villarreal et al., Circulation, Vol. 108, No. 12, pp. 1487-1492 (2003);
(e) restenosis after stenting. For an example of a model useful to demonstrate the treatment of angioplasty, see Huang et al., Heart, Vol. 90, pp. 195-199 (2004);
(f) peripheral vascular disease (PVD) including peripheral artery disease (PAD) and peripheral venous disorders;
(g) coronary arterial disease (CAD). For an example of a model useful to demonstrate the treatment of CAD, see Gerrity et al., Diabetes, Vol. 50, No. 7, pp. 1654-1655 (2001);
(h) morbidity and mortality;
(i) cerebrovascular diseases. For an example of a model useful to demonstrate the treatment of this indication, see Park et al. (2002), *supra;*
(j) metabolic disorder (Syndrome X). See Wang et al., Circulation, Vol. 107, pp. 1923-1929 (2003);
(k) atrial fibrillation (AF);
(l) renoprotection and reduction of proteinuria;
(m) renal failure, e.g., chronic renal failure;
(n) glomerulonephritis (may be associated with the nephrotic syndrome, a high blood pressure and a decreased renal function), focal, segmental glomerulonephritis and minimal change nephropathy;
(o) nephrotic syndrome and renal fibrosis;
(p) acute interstitial nephritis (AIN), acute tubular nephritis (ATN) and acute tubulo-interstitial nephritis;
(q) PKD. See Martinez et al., Am J Kidney Dis, Vol. 29, pp. 435-444 (1997);
(r) vascular inflammation;
(s) rennin secreting tumors;
(t) migraine headaches;
(u) vasculitides; and
(v) closure and restenosis of dialysis access grafts,
comprising administering to a warm-blooded animal an effective amount of a renin inhibitor or a pharmaceutically acceptable salt thereof.

A further object of the present invention is to provide therapeutic compositions and method that further aid in stabilizing plaques comprising administering to a warm-blooded animal an effective amount of a renin inhibitor or a pharmaceutically acceptable salt thereof. By plaque stabilization, it is meant the inhibition of plaque passing through a phase in which the lipid core has grown and the fibrous cap is very thin and vulnerable to rupture due to an increase in the density of macrophages.

In a preferred aspect of the present invention, there is provided a method for the prevention of, delay progression to overt to or treatment of a condition or disease selected from diabetes type 2 (associated with or without hypertension), isolated systolic hypertension, endothelial dysfunction (with or without hypertension), survival post-MI, restenosis after stenting, PVD, CAD, morbidity, mortality, cerebrovascular diseases, metabolic disorder (Syndrome X), renoprotection and vascular inflammation.

The prevention of, delay progression to overt to or treatment of a condition or disease which is different from category (b) as described hereinbefore or hereinafter is understood to be effected in patients without or with hypertension.

A rennin inhibitor is a drug that pharmacologically effectively inhibits the enzyme renin resulting in prevention of, delay progression of and treatment of conditions and diseases associated with the inhibition of renin, especially such conditions and diseases as specified hereinbefore and hereinafter.

Renin inhibitors comprise, e.g., peptidic and, preferably, non-peptidic renin inhibitors.

A non-peptidic renin inhibitor is, e.g., ditekiren, terlakiren, zankiren, SPP-1 00 or a compound of formula (I) 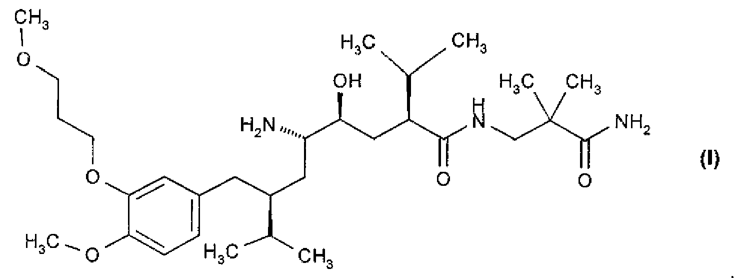 or, in each case, a pharmaceutically acceptable salt thereof.

The renin inhibitor of formula (I), chemically defined as 2(*S*),4(*S*),5(*S*),7(*S*)-*N*-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide, is specifically disclosed in EP 678503 A. Especially preferred is the hemi-fumarate salt thereof.

Non-peptidic renin inhibitor comprise those that are disclosed in WO 97/09311, especially corresponding renin inhibitors as disclosed in the claims and working examples, especially SPP100 of the formula 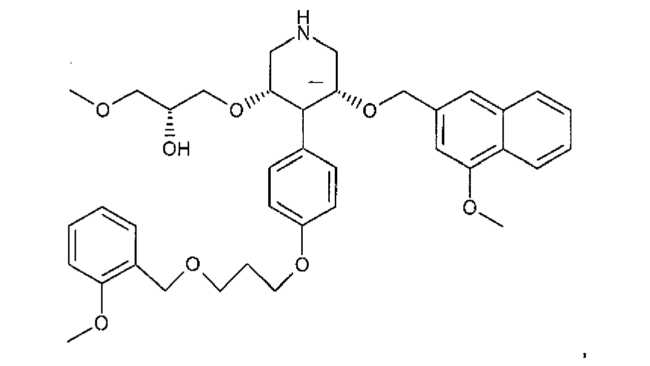 especially and of RO 66-1132 and RO-66-1168 of formula 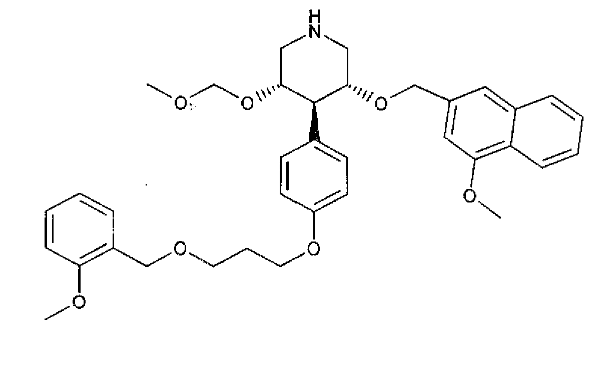 or 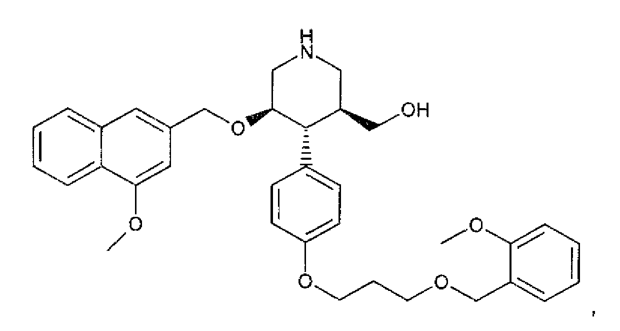 respectively, WO 04/002957, especially those renin inhibitors as disclosed in the working examples and claims. The corresponding subject matter of said WO applications is herein incorporated by reference into the present invention.

The structure of the active agents identified hereinbefore or hereinafter by generic or tradenames may be taken from the actual edition of the standard compendium "The Merck Index" or from databases, e.g., Patent Focus, e.g. IMS Life Cycle - IMS World Publications. The corresponding content thereof is hereby incorporated by reference. Any person skilled in the art is fully enabled to identify the active agents and, based on these references, likewise enabled to manufacture and test the pharmaceutical indications and properties in standard test models, both *in vitro* and *in vivo.*

### DEFINITIONS

Type 2 diabetes mellitus including type 2 diabetes mellitus associated with hypertension is a disease in which the pancreas does not secrete sufficient insulin due to an impairment of pancreatic beta (β)-cell function and/or in which there is to insensitivity to produced insulin (insulin resistance). Typically, the fasting plasma glucose is less than 126 mg/dL, while pre-diabetes is, e.g., a condition which is characterized by one of following conditions: impaired fasting glucose (110-125 mg/dL) and impaired glucose tolerance (fasting glucose levels less than 126 mg/dL and post-prandial glucose level between 140 mg/dL and 199 mg/dL). Type 2 diabetes mellitus can be associated with or without hypertension. Diabetes mellitus occurs frequently, e.g., in African American, Latino/Hispanic American, Native American, Native American, Asian American and Pacific Islanders. Markers of insulin resistance include HbA1C, HOMA IR, measuring collagen fragments, TGF-β in urine, PAI-1 and prorenin.

Severe hypertension is characterized by a characterized by a systolic blood pressure of ≥180 mm Hg and a diastolic blood pressure of ≥110 mm Hg.

PH is a blood vessel disorder of the lung in which the pressure in the pulmonary artery rises above normal level of ≤25/10 (especially primary and secondary PH), e.g., because the small vessels that supply blood to the lungs constrict or tighten up. According to the WHO, PH may be divided into five categories: pulmonary arterial hypertension (PAH), a PH occurring in the absence of a known cause is referred to as primary PH, while secondary PH is caused by a condition selected, e.g., from emphysema; bronchitis; collagen vascular diseases, such as scleroderma, Crest syndrome or systemic lupus erythematosus (SLE); PH associated with disorders of the respiratory system; PH due to chronic thrombotic or embolic disease; PH due to disorders directly affecting the pulmonary blood vessels; and pulmonary venous hypertension (PVH).

Malignant hypertension is usually defined as very high blood pressure with swelling of the optic nerve behind the eye, called papilledema (grade IV Keith-Wagner hypertensive retinopathy). This also includes malignant HTN of childhood.

Isolated systolic hypertension is characterized by a systolic blood pressure of ≥140 mm Hg and a diastolic blood pressure of <90 mm Hg.

Familial dyslipidemic hypertension is characterized by mixed dyslipidemic disorders. Biomarkers include oxidized LDL, HDL, glutathione and homocysteine LPa.

Renovascular hypertension (renal artery stenosis) is a condition where the narrowing of the renal artery is significant which leads to an increase of the blood pressure resulting from signals sent out by the kidneys. Biomarkers include rennin, PRA and prorenin.

Endothelial dysfunction with or without hypertension is a condition in which normal dilation of blood vessels is impaired due to lack of endothelium-derived vasodilators. Biomarkers include CRP, IL6, ET1, BIG ET1, VCAM and ICAM. Survival post-MI biomarkers include BNP and procollagen factors.

Organ/renal/cardiac fibrosis is defined as abnormally high accumulation of collagen and other extracellular matrix proteins due to the enhanced production or decreased degradation of these proteins. Biomarkers include BNP, procollagen factors, LVH, AGE RAGE and CAGE.

Coarctation of aorta is an area of localized narrowing of the large artery (aorta). The narrowing may be caused by a "shelf" tissue inside the blood vessel which reduces its area. Alternatively, it may be caused by underdevelopment of portion of the aorta itself which cases a longer area of reduced diameter.

Restenosis after percutaneous transluminal angioplasty is defined as the closure of an artery following a procedure to open said artery that is fully or partially-blocked by the accumulation of plaque or other disease. Biomarkers include coronary flow reserve.

Peripheral vascular disease (PVD) refers to the damage or dysfunction of peripheral blood vessels. There are two types of peripheral vascular diseases: peripheral arterial disease (PAD) which refers to diseased peripheral arteries and peripheral venous disorders, which can be measured by an ankle brachial index.

PAD is a condition that progressively hardens and narrows arteries due to a gradual buildup of plaque and refers to conditions that effect the blood vessels, such as arteries, veins and capillaries, of the body outside the heart. This is also known as peripheral venous disorder.

Thrombophlebitis is a condition where an obstructing blood clot has been formed causing the surrounding veins to become inflamed.

Varicose veins is a condition where abnormally widened veins are swollen, dark and twisted or contorted. This usually occurs in the legs.

Chronic venous insufficiency is an advanced stage of leg vein disease in which the veins become incompetent causing blood to pool in the legs and feed and sometimes to leak backwards.

Coronary arterial disease (CAD) is a condition that progressively hardens and narrows arteries due to a gradual buildup of plaque and refers to conditions that effect the blood vessels such as arteries within the heart. CAD is peculiar form of atherosclerosis that occurs in the three small arteries supplying the heart muscle with oxygen-rich blood. Biomarkers include CPK and Troponin.

Cerebrovascular diseases comprise stroke conditions, such as embolic and thrombotic stroke; large vessel thrombosis and small vessel disease; and hemorrhagic stroke.

Embolic stroke is characterized by the formation of blood clots, e.g., in the heart, when clots travel down through the bloodstream in the brain. This may lead to a blockade of small blood vessels and causing a stroke.

Thrombotic stroke is a condition where the blood flow is impaired because of a blockade to one or more of the arteries supplying blood to the brain. This process normally leads to thrombosis causing thrombotic strokes. Biomarkers include PAI 1, TPA and platelet function.

Metabolic disorder (Syndrome X): Among various definitions of metabolic syndrome that are known three of them are of particular relevance. Metabolic syndrome is characterized by three or more of the following criteria:
1. Abdominal obesity: waist circumference >102 cm in men and >88 cm in women
2. Hypertriglyceridemia: >150 mg/dL (1.695 mmol/L)
3. Low HDL cholesterol: <40 mg/dL (1.036 mmol/L) in men and <50 mg/dL (1.295 mmol/L) in women
4. High blood pressure: >130/85 mm Hg
5. High-fasting glucose: >110 mg/dL (>6.1 mmol/L)

Metabolic syndrome can also be characterized by three or more of the following criteria: triglycerides >150 mg/dL, systolic blood pressure (BP) ≥130 mm Hg or diastolic BP ≥85 mm Hg or on anti-hypertensive treatment, high-density lipoprotein cholesterol <40 mg/dL, fasting blood sugar (FBS) >110 mg/dL, and a body mass index (BMI) >28.8 k/m².

Metabolic syndrome can also be characterized by diabetes, impaired glucose tolerance, impaired fasting glucose, or insulin resistance plus two or more of the following abnormalities:
1. High blood pressure: ≥160/90 mm Hg
2. Hyperlipidemia: triglyceride concentration ≥150 mg/dL (1.695 mmol/L) and/or HDL cholesterol <35 mg/dL (0.9 mmol/L) in men and <39 mg/dL (1.0 mmol/L) in women
3. Central obesity: waist-to-hip ratio of >0.90 in men or >0.85 in women and/or BMI >30 kg/m²
4. Microalbuminuria: urinary albumin excretion rate ≥20 µg/min. or an albumin-to-creatinine ratio ≥20 mg/g. Biomarkers include proteinuria, TGF-β, TNF-α and adiponectin.

Biomarkers include LDL, HDL and all the endothelial dysfunction markers.

AF is a type of irregular or "racing" heartbeat that may cause blood to collect in the heart and potentially form a clot which may travel to the brain and can cause a stroke.

Organ protection is the prevention of loss of function of the restoration of impaired function of a bodily organ.

Renoprotection is reduction of proteinuria. Biomarkers include, collagen fragments and TGF-β in urine.

Renal failure, e.g., chronic renal failure; is characterized, e.g., by proteinuria and/or slight elevation of plasma creatinine concentration (106-177 mmol/L corresponding to 1.2-2.0 mg/dL).

Glomerulonephritis may be associated with the nephrotic syndrome, a high blood pressure and a decreased renal function; focal, segmental glomerulonephritis; minimal change nephropathy, Lupus nephritis, post-streptococal GN and IgA nephropathy.

Nephrotic syndrome is a compilation of conditions including massive proteinuria, edema and CNS irregularities. Biomarkers include urinary protein excretion.

Plaque stabilization means rendering a plaque less dangerous by preventing, fibrous cap thinning/rupture, smooth muscle cell loss and inflammatory cell accumulation.

Renal fibrosis is an abnormal accumulation of collagen and other extracellular matrix proteins, leading to loss of renal function. Biomarkers include collagen fragments and TGF-β in urine.

End-stage renal disease (ESRD) is loss of renal function to the extent that dialysis or renal replacement is needed. Biomarkers include glomerular filtration rate and creatinine clearance.

Polycystic kidney disease (PKD) is a genetic disorder characterized by the growth of numerous cysts in the kidney. PKD cysts can slowly reduce much of the mass of kidneys reducing kidney function and leading to kidney failure. PKD may be classified as two major inherited forms of PKD which are autosomal dominant PKD and autosomal recessive PKD, while the non-inherited PKD may be called acquired cystic kidney disease. Biomarkers include reduction of renal cysts by non-invasive imaging.

Obesity is an overweight condition defined by BMI of >30.

The term "prevention" means prophylactic administration to healthy patients to prevent the outbreak of the conditions mentioned herein. Moreover, the term "prevention" means prophylactic administration to patients being in a pre-stage of the conditions, to be treated.

The term "delay progression to overt to", as used herein, means administration to patients being in a pre-stage of the condition to be treated in which patients a pre-form of the corresponding condition is diagnosed.

The term "treatment" is understood the management and care of a patient for the purpose of combating the disease, condition or disorder.

An "effective amount" shall mean that amount of compound that will elicit the biological or medical response of a tissue, system or animal (including man) that is being sought by a researcher or clinician.

The terms "warm-blooded animal or patient" are used interchangeably herein and include, but are not limited to, humans, dogs, cats, horses, pigs, cows, monkeys, rabbits, mice and laboratory animals. The preferred mammals are humans.

A "pharmaceutically acceptable salt" refers to a non-toxic salt commonly used in the pharmaceutical industry, which are prepared by methods well-known in the art.

Surprisingly, the present invention has a longer duration of action than ACE or ARB's while maintaining the same efficacy. Moreover, renin is a direct vasodilator and has direct profibrotic effects. By blocking renin activity one would expect anti-inflammatory and antifibrotic effects in addition to what is seen with ACE and ARBs due to additive effect of RI on ANGII and rennin blockade. Further, renin blockade effects ANG4 and ANG1-7. ANG 1-7 is suspected to have beneficial effects which effects inflammation, thrombosis, fibrosis and cell proliferation.

All the more surprising is the experimental finding that the combined administration of the renin inhibitor of formula (I) or a salt thereof with a therapeutic agent selected from the group consisting of (i)-(xiv), as defined below, results not only in a beneficial, especially a synergistic, therapeutic effect or a potentiation of at least one of the combination partners, but also in additional benefits resulting from the combined treatment and further surprising beneficial effects compared to a monotherapy applying only one of the pharmaceutically active compounds used in the combinations disclosed herein.

The invention similarly relates to combinations, e.g., pharmaceutical combinations, containing a renin inhibitor of the present invention or, in each case, a pharmaceutically acceptable salt thereof in combination with at least one active principle, or in each case, a pharmaceutically acceptable salt thereof.

The combination may be made, e.g., with the following compositions, selected from the group consisting of:
(i) an angiotensin II receptor antagonist or a pharmaceutically acceptable salt thereof;
(ii) ACE inhibitor or a pharmaceutically acceptable salt thereof;
(iii) CCB or a pharmaceutically acceptable salt thereof;
(iv) HMG-Co-A reductase inhibitor or a pharmaceutically acceptable salt thereof;
(v) aldosterone synthase inhibitor or a pharmaceutically acceptable salt thereof;
(vi) aldosterone antagonist or a pharmaceutically acceptable salt thereof;
(vii) dual ACE/NEP inhibitor or a pharmaceutically acceptable salt thereof;
(viii) β-blocker or a pharmaceutically acceptable salt thereof;
(ix) endothelin (ET) antagonist or a pharmaceutically acceptable salt thereof;
(x) diuretic or a pharmaceutically acceptable salt thereof;
(xi) oral hypoglycaemic agent or a pharmaceutically acceptable salt thereof;
(xii) a Mrp2 inhibitor;
(xiii) furosemide or a pharmaceutically acceptable salt thereof; and
(xiv) Gleevec or a pharmaceutically acceptable salt thereof.

The combination according to the present invention can be used, e.g., for the prevention of, delay progression to overt to or treatment of a condition or disease selected from:
(a) diabetes type 2 (associated with or without hypertension);
(b) severe hypertension, PH, malignant hypertension, isolated systolic hypertension and familial dyslipidemic hypertension;
(c) endothelial dysfunction (with or without hypertension);
(d) survival post-MI, increase of formation of collagen and coarctation of aorta;
(e) restenosis after percutaneous transluminal angioplasty
(f) PVD, including PAD and peripheral venous disorders;
(g) CAD;
(h) morbidity and mortality;
(i) cerebrovascular diseases;
(j) metabolic disorder (Syndrome X);
(k) AF;
(l) organ protection;
(m) renoprotection;
(n) renal failure, e.g., chronic renal failure;
(o) glomerulonephritis (may be associated with the nephritic syndrome, a high blood pressure and a decreased renal function), focal, segmental glomerulonephritis and minimal change nephropathy;
(p) nephrotic syndrome and renal fibrosis;
(q) AIN, ATN and acute tubulo-interstitial nephritis;
(r) ESRD;
(s) PKD;
(t) vascular inflammation;
(u) obesity;
(v) migraine headaches
(w) renin secreting tumors; and
(x) vasculitides.

The combination according to the present invention may be used in a method of stabilizing plaques comprising administering to a warm-blooded animal an effective amount of a combination of the invention or pharmaceutically acceptable salts thereof.

The combination according to the present invention comprising a renin inhibitor or a pharmaceutically acceptable salt thereof can be administered by various routes of administration. Each agent can be tested over a wide-range of dosages to determine the optimal drug level for each agent in combination to elicit the maximal response. For these studies, it is preferred to use treatment groups consisting of at least 6 animals per group. Each study is best performed in which the effects of the combination treatment group are determined at the same time as the individual components are evaluated. Although drug effects may be observed with acute administration (such as 1 day), it is preferable to observe responses in a chronic setting as shown below in which experiments were done over a two to three week observation period. The long-term study is of sufficient duration to allow for the full development of compensatory responses to occur and therefore, the observed effect will most likely depict the actual responses of the test system representing sustained or persistent effects.

AT₁-receptor antagonists (also called angiotensin II receptor antagonists) are understood to be those active ingredients that bind to the AT₁-receptor subtype of angiotensin II receptor but do not result in activation of the receptor. As a consequence of the inhibition of the AT₁-receptor, these antagonists can, e.g., be employed as anti-hypertensives or for treating congestive heart failure.

The class of AT₁-receptor antagonists comprises compounds having differing structural features, essentially preferred are the non-peptidic ones. For example, mention may be made of the compounds that are selected from the group consisting of valsartan (cf. EP 443983), losartan (cf. EP253310), candesartan (cf. 459136), eprosartan (cf. EP 403159), irbesartan (cf. EP454511), olmesartan (cf. EP 503785), tasosartan (cf. EP539086), telmisartan (cf. EP 522314), the compound with the designation E-1477 of the formula 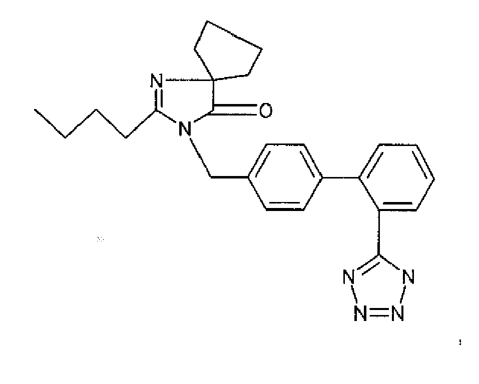 the compound with the designation SC-52458 of the formula 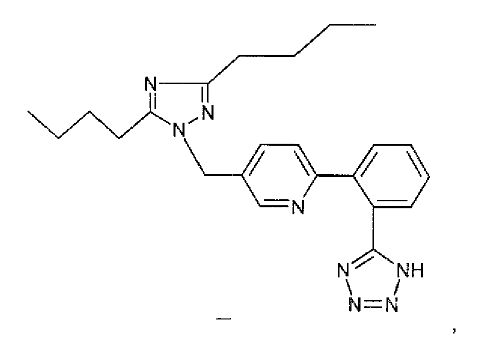 and the compound with the designation ZD-8731 of the formula 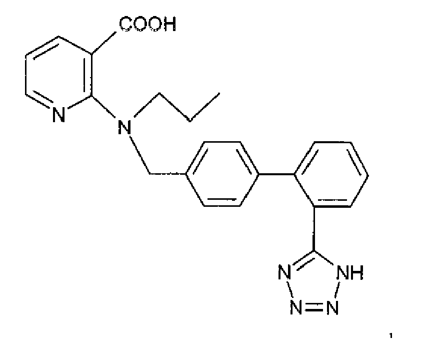 or, in each case, a pharmaceutically acceptable salt thereof.

Preferred AT₁-receptor antagonist are those agents that have been marketed, most preferred is valsartan or a pharmaceutically acceptable salt thereof.

HMG-Co-A reductase inhibitors (also called β-hydroxy-β-methylglutaryl-co-enzyme-A reductase inhibitors) are understood to be those active agents that may be used to lower the lipid levels including cholesterol in blood.

The class of HMG-Co-A reductase inhibitors comprises compounds having differing structural features. For example, mention may be made of the compounds that are selected from the group consisting of atorvastatin, cerivastatin, compactin, dalvastatin, dihydrocompactin, fluindostatin, fluvastatin, lovastatin, pitavastatin, mevastatin, pravastatin, rivastatin, simvastatin, rosuvastatin and velostatin, or, in each case, a pharmaceutically acceptable salt thereof.

Preferred FIMG-Co-A reductase inhibitors are those agents which have been marketed, most preferred is fluvastatin and pitavastatin or, in each case, a pharmaceutically acceptable salt thereof.

The interruption of the enzymatic degradation of angiotensin I to angiotensin II with so-called ACE inhibitors is a successful variant for the regulation of blood pressure and thus also makes available a therapeutic method for the treatment of congestive heart failure.

The class of ACE inhibitors comprises compounds having differing structural features. For example, mention may be made of the compounds which are selected from the group consisting alacepril, benazepril, benazeprilat, captopril, ceronapril, cilazapril, delapril, enalapril, enaprilat, fosinopril, imidapril, lisinopril, moveltopril, perindopril, quinapril, ramipril, spirapril, temocapril and trandolapril, or, in each case, a pharmaceutically acceptable salt thereof.

Preferred ACE inhibitors are those agents that have been marketed, most preferred are benazepril and enalapril.

The class of CCBs essentially comprises dihydropyridines (DHPs) and non-DHPs, such as diltiazem-type and verapamil-type CCBs.

A CCB useful in said combination is preferably a DHP representative selected from the group consisting of amlodipine, felodipine, ryosidine, isradipine, lacidipine, nicardipine, nifedipine, niguldipine, niludipine, nimodipine, nisoldipine, nitrendipine and nivaldipine, and is preferably a non-DHP representative selected from the group consisting of flunarizine, prenylamine, diltiazem, fendiline, gallopamil, mibefradil, anipamil, tiapamil and verapamil, and in each case, a pharmaceutically acceptable salt thereof. All these CCBs are therapeutically used, e.g., as anti-hypertensive, anti-angina pectoris or anti-arrhythmic drugs.

Preferred CCBs comprise amlodipine, diltiazem, isradipine, nicardipine, nifedipine, nimodipine, nisoldipine, nitrendipine and verapamil, or, e.g., dependent on the specific CCB, a pharmaceutically acceptable salt thereof. Especially preferred as DHP is amlodipine or a pharmaceutically acceptable salt, especially the besylate, thereof. An especially preferred representative of non-DHPs is verapamil or a pharmaceutically acceptable salt, especially the hydrochloride, thereof.

Aldosterone synthase is an enzyme that converts corticosterone to aldosterone by hydroxylating corticosterone to form 18-OH-corticosterone and 18-OH-corticosterone to aldosterone. The class of aldosterone synthase inhibitors is known to be applied for the treatment of hypertension and primary aldosteronism comprises both steroidal and non-steroidal aldosterone synthase inhibitors, the later being most preferred.

Preference is given to commercial-available aldosterone synthase inhibitors or those aldosterone synthase inhibitors that have been approved by the health authorities.

The class of aldosterone synthase inhibitors comprises compounds having differing structural features. For example, mention may be made-of the compounds which are selected from the group consisting of the non-steroidal aromatase inhibitors anastrozole, fadrozole (including the (+)-enantiomer thereof), as well as the steroidal aromatase inhibitor exemestane, or, in each case where applicable, a pharmaceutically acceptable salt thereof.

The most preferred non-steroidal aldosterone synthase inhibitor is the (+)-enantiomer of the (U.S. Patent Nos. 4,617,307 and 4,889,861), or a pharmaceutically acceptable salt thereof, e.g., the hydrochloride of fadrozole of formula 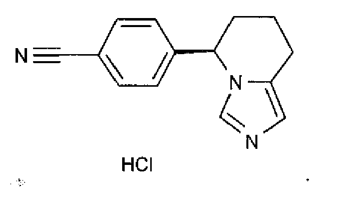 A preferred steroidal aldosterone antagonist is splarenone of the formula 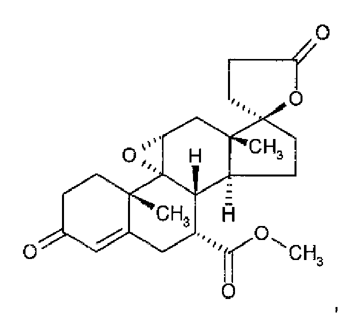 or spironolactone.

A preferred dual ACE/NEP inhibitor is, e.g., omapatrilate (cf. EP 629627), fasidotril or fasidotrilate, or Z 13752A (cf. WO 97/24342) or, if appropriable, a pharmaceutically acceptable salt thereof.

β-blockers suitable for use in the present invention include β-adrenergic blocking agents (β-blockers) which compete with epinephrine for β-adrenergic receptors and interfere with the action of epinephrine. Preferably, the β-blockers are selective for the β-adrenergic receptor as compared to the alpha (α)-adrenergic receptors, and so do not have a significant α-blocking effect. Suitable β-blockers include compounds selected from acebutolol, atenolol, betaxolol, bisoprolol, carteolol, carvedilol, esmolol, labetalol, metoprolol, nadolol, oxprenolol, penbutolol, pindolol, propranolol, sotalol and timolol. Where the β-blocker is an acid or base or otherwise capable of forming pharmaceutically acceptable salts or prodrugs, these forms are considered to be encompassed herein, and it is understood that the compounds may be administered in free form or in the form of a pharmaceutically acceptable salt or a prodrug, such as a physiologically hydrolizable and acceptable ester. For example, metoprolol is suitably administered as its tartrate salt, propranolol is suitably administered as the hydrochloride salt, and so forth.

ET is a highly-potent, vasoconstrictor peptide synthesized and released by the vascular endothelium. ET exists in three isoforms (ET-1, ET-2 and ET-3). ET shall mean any or all other isoforms of ET. Elevated levels of ET have been reported in plasma from patients with, e.g., essential hypertension. ET receptor antagonist can be used to inhibit the vasoconstrictive effects induced by ET.

A preferred ET antagonist is, e.g., bosentan (cf. EP 526708 A), enrasentan (cf. WO 94/25013), atrasentan (cf. WO 96/06095), especially atrasentan hydrochloride, darusentan (cf. EP 785926 A), BMS 193884 (cf. EP 702012 A ), sitaxentan (cf. U.S. Patent No. 5,594,021), especially sitaxsentan sodium, YM 598 (cf. EP 882719 A), S 0139 (cf. WO 97/27314), J 104132 (cf. EP 714897 A or WO 97/37665), furthermore, tezosentan (cf. WO 96/19459), or in each case, a pharmaceutically acceptable salt thereof.

A diuretic is, e.g., a thiazide derivative selected from the group consisting of chlorothiazide, hydrochlorothiazide, methylclothiazide, amiloride, triamterene and chlorothalidon. The most preferred is hydrochlorothiazide.

Preferably, the jointly therapeutically effective amounts of the active agents according to the combination of the present invention can be administered simultaneously or sequentially in any order, separately or in a fixed combination.

The pharmaceutical composition according to the present invention as described hereinbefore and hereinafter may be used for simultaneous use or sequential use in any order, for separate use or as a fixed combination.

The corresponding active ingredients or a pharmaceutically acceptable salts thereof may also be used in form of a solvate, such as a hydrate or including other solvents, used for crystallization.

The compounds to be combined can be present as pharmaceutically acceptable salts. If these compounds have, e.g., at least one basic center, they can form acid addition salts. Corresponding acid addition salts can also be formed having, if desired, an additionally present basic center. The compounds having an acid group, e.g., COOH, can also form salts with bases.

In a variation thereof, the present invention likewise relates to a "kit-of-parts", e.g., in the sense that the components to be combined according to the present invention can be dosed independently or by use of different fixed combinations with distinguished amounts of the components, i.e., simultaneously or at different time points. The parts of the kit of parts can then, e.g., be administered simultaneously or chronologically staggered, that is at different time points and with equal or different time intervals for any part of the kit of parts. Preferably, the time intervals are chosen such that the effect on the treated disease or condition in the combined use of the parts is larger than the effect that would be obtained by use of only any one of the components.

The invention furthermore relates to a commercial package comprising the combination according to the present invention together with instructions for simultaneous, separate or sequential use.

Dosaging may depend on various factors, such as mode of application, species, age and/or individual condition. For oral application, the doses to be administered daily are between 10 mg to 1 g.

The term "synergistic", as used herein, means that the effect achieved with the methods and compositions of the present invention is greater than the sum of the effects that result from individual methods and compositions comprising the active ingredients of this invention separately.

The person skilled in the pertinent art is fully enabled to select a relevant and standard animal test model to prove the hereinbefore and hereinafter indicated therapeutic indications and beneficial effects.

These pharmaceutical preparations are for enteral, such as oral, and also rectal or parenteral, administration to homeotherms, with the preparations comprising the pharmacological active compound either alone or together with customary pharmaceutical auxiliary substances. For example, the pharmaceutical preparations consist of from about 0.1-90%, preferably of from about 1% to about 80%, of the active compound. Pharmaceutical preparations for enteral or parenteral, and also for ocular, administration are, e.g., in unit dose forms, such as coated tablets, tablets, capsules or suppositories and also ampoules. These are prepared in a manner that is known per se, e.g., using conventional mixing, granulation, coating, solubulizing or lyophilizing processes. Thus, pharmaceutical preparations for oral use can be obtained by combining the active compound with solid excipients, if desired granulating a mixture which has been obtained, and, if required or necessary, processing the mixture or granulate into tablets or coated tablet cores after having added suitable auxiliary substances.

The dosage of the active compound can depend on a variety of factors, such as mode of administration, homeothermic species, age and/or individual condition.

Preferred dosages for the active ingredients of the pharmaceutical combination according to the present invention are therapeutically effective dosages, especially those which are commercially-available.

Normally, in the case of oral administration, an approximate daily dose of from about 1 mg to about 360 mg is to be estimated, e.g., for a patient of approximately 75 kg in weight.

The dosage of the active compound can depend on a variety of factors, such as mode of administration, homeothermic species, age and/or individual condition.

The pharmaceutical preparation will be supplied in the form of suitable dosage unit form, e.g., a capsule or tablet, and comprising an amount, being together with the further component(s) jointly effective.

The doses of renin inhibitor of formula (I) to be administered to warm-blooded animals, e.g., human beings, of, e.g., approximately 70 kg body weight, especially the doses effective in the inhibition of the enzyme renin, e.g., in lowering blood pressure and/or in improving the symptoms of glaucoma, are from approximately 3 mg to approximately 3 g, preferably from approximately 10 mg to approximately 1 g, e.g., approximately from 20-200 mg/person/day, divided preferably into 1-4 single doses which may, e.g., be of the same size. Usually, children receive about half of the adult dose. The dose necessary for each individual can be monitored, e.g., by measuring the serum concentration of the active ingredient, and adjusted to an optimum level. Single doses comprise, e.g., 75 mg, 150 mg or 300 mg per adult patient.

Valsartan, as a representative of the class of AT₁-receptor antagonists, will be supplied in the form of suitable dosage unit form, e.g., a capsule or tablet, and comprising a therapeutically effective amount, e.g., from about 20 mg to about 320 mg, of valsartan which may be applied to patients. The application of the active ingredient may occur up to three times a day (t.i.d.), starting, e.g., with a daily dose of 20 mg or 40 mg of valsartan, increasing via 80 mg daily and further to 160 mg daily up to 320 mg daily. Preferably, valsartan is applied twice a day (b.i.d.) with a dose of 80 mg or 160 mg, respectively, each. Corresponding doses may be taken, e.g., in the morning, at mid-day or in the evening. Preferred is b.i.d. administration.

In case of HMG-Co-A reductase inhibitors, preferred dosage unit forms of HMG-Co-A reductase inhibitors are, e.g., tablets or capsules comprising, e.g., from about 5 mg to about 120 mg, preferably, when using fluvastatin, e.g., 20 mg, 40 mg or 80 mg (equivalent to the free acid) of fluvastatin, e.g., administered once a day.

In case of ACE inhibitors, preferred dosage unit forms of ACE inhibitors are, e.g., tablets or capsules comprising, e.g., from about 5 mg to about 20 mg, preferably 5 mg, 10 mg, 20 mg or 40 mg of benazepril; from about 6.5-100 mg, preferably 6.25 mg, 12.5 mg, 25 mg, 50 mg, 75 mg or 100 mg of captopril; from about 2.5 mg to about 20 mg, preferably 2.5 mg, 5 mg, 10 mg or 20 mg of enalapril; from about 10 mg to about 20 mg, preferably 10 mg or 20 mg of fosinopril; from about 2.5 mg to about 4 mg, preferably 2 mg or 4 mg of perindopril; from about 5 mg to about 20 mg, preferably 5 mg, 10 mg or 20 mg of quinapril; or from about 1.25 mg to about 5 mg, preferably 1.25 mg, 2.5 mg, or 5 mg of ramipril. Preferred is t.i.d. administration.

For example, suitable daily dosages of β-blockers for adults of the following compounds for oral administration are as indicated: acebutol - 200-1200 mg; atenolol - 25-100 mg; betaxofol - 10-20 mg; bisoprolol - 5-10 mg; carteolol - 2.5-10 mg; labetalol - 100-1,800 mg; metoprolol - 50-450 mg; nadolol - 40-240 mg; oxprenol - 60-480 mg; penbutolol - 20-80 mg; pindolol - 10-60 mg; propranolol - 40-320 mg or 60-320 mg for long-acting formulation); sotalol - 160-320 mg; timolol - 20-60 mg. Especially preferred β-blockers for use in the present invention are atenolol, metoprolol and propranolol.

Especially preferred are low-dose combinations.

## Claims

1. A method for the prevention of, delay progression to or treatment of a condition or disease selected from diabetes type 2 (associated with or without hypertension), severe hypertension, pulmonary hypertension (PH), malignant hypertension, isolated systolic hypertension, familial dyslipidemic hypertension, endothelial dysfunction (with or without hypertension), survival post-myocardial infarction (MI), increase of formation of collagen and other extracellular matrix proteins, restenosis after stenting, peripheral vascular disease (PVD) including peripheral artery disease (PAD) and peripheral venous disorders, coronary arterial disease (CAD), morbidity, mortality, cerebrovascular diseases, metabolic disorder (Syndrome X), atrial fibrillation (AF), renoprotection, reduction of proteinuria, renal failure, glomerulonephritis, nephrotic syndrome, renal fibrosis, acute interstitial nephritis (AIN), acute tubular nephritis (ATN), acute tubulo-interstitial nephritis, polycystic kidney disease (PKD), vascular inflammation, renin secreting tumors, vasculitides or closure, restenosis of dialysis access grafts, comprising administering to a warm-blooded animal an effective amount of a renin inhibitor or a pharmaceutically acceptable salt thereof.

2. A method of aiding plaque stabilization comprising administering to a warm-blooded animal an effective amount of a renin inhibitor or a pharmaceutically acceptable salt thereof.

3. The method of Claim 1, wherein the renin inhibitor is a compound of formula (I) 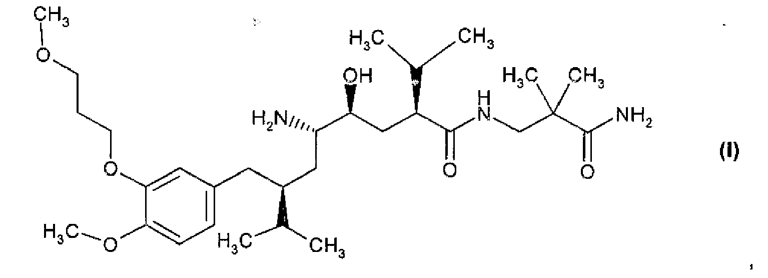 or a pharmaceutically acceptable salt thereof.

4. The method of Claim 2, wherein the renin inhibitor is a compound of formula (I) 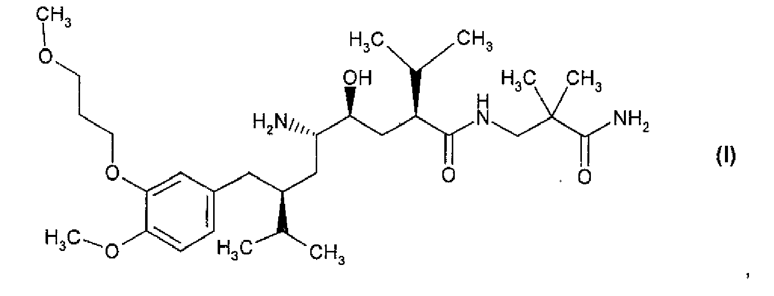 or a pharmaceutically acceptable salt thereof.

5. A pharmaceutical composition comprising a renin inhibitor in combination and at least one additional active agent selected from the group consisting of:
(i) an angiotensin II receptor antagonist or a pharmaceutically acceptable salt thereof;
(ii) ACE inhibitor or a pharmaceutically acceptable salt thereof;
(iii) CCB or a pharmaceutically acceptable salt thereof;
(iv) HMG-Co-A reductase inhibitor or a pharmaceutically acceptable salt thereof;
(v) aldosterone synthase inhibitor or a pharmaceutically acceptable salt thereof;
(vi) aldosterone antagonist or a pharmaceutically acceptable salt thereof;
(vii) dual ACE/NEP inhibitor or a pharmaceutically acceptable salt thereof;
(viii) β-blocker or a pharmaceutically acceptable salt thereof;
(ix) endothelin antagonist or a pharmaceutically acceptable salt thereof;
(x) diuretic or a pharmaceutically acceptable salt thereof;
(xi) oral hypoglycaemic agent or a pharmaceutically acceptable salt thereof;
(xii) Mrp2 inhibitor;
(xiii) furosemide or a pharmaceutically acceptable salt thereof; and
(xiv) Gleevec or a pharmaceutically acceptable salt thereof.

6. The pharmaceutical composition of Claim 5, wherein the rennin inhibitor is a compound of formula (I) or a pharmaceutically acceptable salt thereof.

7. A method for the prevention of, delay progression to overt to or treatment of a condition or disease selected from:
(a) diabetes type 2 (associated with or without hypertension);
(b) severe hypertension, PH, malignant hypertension, isolated systolic hypertension and familial dyslipidemic hypertension;
(c) endothelial dysfunction (with or without hypertension);
(d) survival post-MI, increase of formation of collagen and coarctation of aorta;
(e) restenosis after percutaneous transluminal angioplasty
(f) PVD, including PAD and peripheral venous disorders;
(g) CAD;
(h) morbidity and mortality;
(i) cerebrovascular diseases;
(j) metabolic disorder (Syndrome X);
(k) AF;
(l) organ protection;
(m) renoprotection;
(n) renal failure, e.g., chronic renal failure;
(o) glomerulonephritis (may be associated with the nephritic syndrome, a high blood pressure and a decreased renal function), focal, segmental glomerulonephritis and minimal change nephropathy;
(p) nephrotic syndrome and renal fibrosis;
(q) AIN, ATN and acute tubulo-inerstitial nephritis;
(r) end-stage renal disease (ESRD);
(s) PKD;
(t) vascular inflammation;
(u) obesity;
(v) migraine headaches
(w) renin secreting tumors; and
(x) vasculitides,
comprising administering to a warm-blooded animal an effective amount of the pharmaceutical composition of Claim 5.

8. The method of Claim 7, wherein the rennin inhibitor is a compound of formula (I) or a pharmaceutically acceptable salt thereof.

9. A method of aiding plaque stabilization comprising administering to a warm-blooded animal an effective amount of the pharmaceutical composition of Claim 5.

10. The method of Claim 9, wherein the renin inhibitor is a compound of formula (I) 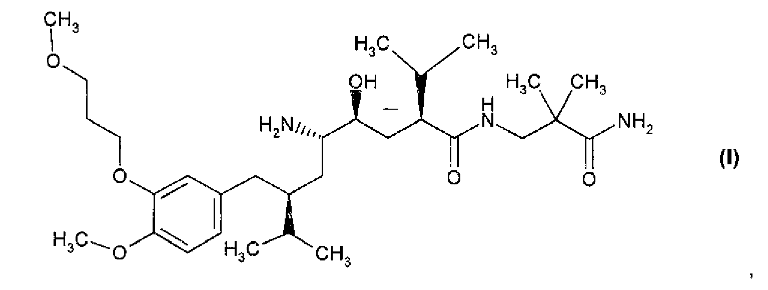 or a pharmaceutically acceptable salt thereof.
